Europäisches Patentamt

European Patent Office

Office européen des brevets

(11) Numéro de publication: **0 408 444 A2**

# DEMANDE DE BREVET EUROPEEN

(21) Numéro de dépôt: 90401987.4

(51) Int. Cl.⁵: **C08B 37/00, C12P 21/00**

(22) Date de dépôt: **10.07.90**

(30) Priorité: **11.07.89 FR 8909305**

(43) Date de publication de la demande:
**16.01.91 Bulletin 91/03**

(84) Etats contractants désignés:
**AT BE CH DE DK ES GB GR IT LI LU NL SE**

(71) Demandeur: **ROUSSEL-UCLAF**
**35, boulevard des Invalides**
**F-75007 Paris(FR)**

(72) Inventeur: **Smets, Pierre**
**137, Avenue du Maréchal Leclerc**
**F-78670 Villennes Sur Seine(FR)**
Inventeur: **Zalisz, René**
**20, rue Delattre de Tassigny**
**F-95180 Menucourt(FR)**

(74) Mandataire: **Vieillefosse, Jean-Claude et al**
**Roussel-Uclaf 111, route de Noisy B.P. 9**
**F-93230 Romainville(FR)**

(54) Galactanne extrait de Klebsiella, procédé d'obtention et application à titre de médicament.

(57) Le galactanne extrait de Klebsiella, caractérisé en ce qu'il est essentiellement constitué d'oses neutres, procédé d'obtention et application à titre de médicament.

EP 0 408 444 A2

# GALACTANNE EXTRAIT DE KLEBSIELLA, PROCÉDÉ D'OBTENTION ET APPLICATION À TITRE DE MÉDI-CAMENT.

La présente invention concerne du galactanne à faible poids moléculaire extrait de Klebsiella, son procédé d'obtention, son application à titre de médicament, en particulier comme immunostimulant.

La demande de brevet français FR 2 574 429 décrit des acylglycannes extraits de Klebsiella constitues d'environ 80% d'oses neutres, 20% de lipides, moins de 2% de protéines, ayant un poids moléculaire d'environ 12500 et renfermant un enchaînement de galactoses liés en 1-3. Ils sont doués de propriétés antiallergiques et immunomodulatrices.

La présente invention concerne du galactanne extrait de Klebsiella, caractérisé en ce qu'il est essentiellement constitué d'oses neutres.

La composition en oses neutres est déterminée par chromatographie en phase gazeuse après méthanolyse selon la méthode de ZANETTA J. Chromato. (1972) 69 , p. 291 ou la méthode de KAMERLING (Biochem J. (1975), 151 p. 491).

La présente invention concerne plus particulièrement du galactanne, caractérisé en ce qu'il est constitué d'un enchaînement linéaire de galactoses en 1-3 et qu'il a un poids moléculaire compris entre 4000 et 10000.

Ces polysaccharides sont exempts de lipides et de protéines.

La composition en galactose est déterminée par chromatographie en phase gazeuse, après réduction et méthanolyse.

Les lipides sont dosés par chromatographie en phase gazeuse après méthanolyse.

Les protéines sont dosées par la méthode de LOWRY (J.B.C. (1951), 193 , p. 265-273).

La séquence d'enchaînement des galactoses est déterminée par les méthodes classiques de méthylation, analyse par chromatographie en phase gazeuse couplée à la spectométrie de masse, oxydation périodique, RMN de $^1$H et $^{13}$C. Les résultats montrent que le galactanne est formé d'une chaîne linéaire de résidus de galactoses reliés par des liaisons alpha (1-3) 50% et béta (1-3) 50%, avec un motif de répétition constitué de 8 résidus galactopyranose et 1 résidu galactofuranose.

Le galactanne se présente sous forme d'une poudre blanche lyophilisée hydrosoluble.

Le galactanne de l'invention peut être extrait de différentes espèces de Klebsiella. On retient particulièrement celui qui provient de Klebsiella pneumoniae et tout particulièrement celui qui provient de la souche déposée à l'Institut Pasteur à Paris ou Collection Nationale de Culture de Microorganismes sous les numéros 52145 et I-163, et à la National Culture Type Collection sous le n° 5055. Cette souche est depuis longtemps commercialisée et librement disponible auprès du public, notamment auprès de l'Institut Pasteur à Paris sous le n° 52145 ; cette souche a de plus été déposée par la demanderesse le 25 juin 1981 sous le n° I-163 à l'Institut Pasteur à Paris (CNCM) Collection Nationale de Culture de Microorganismes.

La présente invention a également pour objet un procédé d'obtention du galactanne, tel que défini ci-dessus, ledit procédé étant caractérisé en ce que l'on traite un acylglycanne extrait de Klebsiella, essentiellement constitué d'environ 80% d'oses neutres, 20% de lipides, moins de 2% de protéines et ayant un poids moléculaire d'environ 12500, par hydrolyse douce, chromatographie à haute performance sur échangeur d'anions, récupère la fraction non retenue, soumet à une filtration sur gel, récupère la fraction contenant le galactanne de poids moléculaire compris entre 4000 et 10000.

Le galactanne de Klebsiella de départ peut être obtenu à partir d'un extrait bactérien hydrosoluble de Klebsiella par chauffage puis fractionnement chromatographique. De telles préparations ont déjà été décrites dans le brevet français FR 2 574 429. L'extrait bactérien hydrosoluble de Klebsiella a été préparé selon le procédé décrit dans le brevet EP 49182.

Selon des conditions préférentielles de mise en oeuvre du procédé selon l'invention :

- L'hydrolyse acide douce de l'acylglycanne est effectuée dans une solution à 1% d'acide acétique par chauffage à 100°C pendant 90 minutes ; le précipité formé, qui renferme la fraction lipidique, est éliminé par centrifugation, par exemple 30 minutes à 2000 g. Le surnageant qui renferme le galactanne est recueilli et peut être lyophilisé.

- Le galactanne est alors séparé du surnageant par fractionnement chromatographique. On recueille la fraction constituée d'oses neutres.

Ce fractionnement peut être effectué par chromatographie sur échangeurs d'anions, de préférence par chromatographie haute performance, par exemple sur Magnum 9SAX Whatman. La chromatographie à haute performance sur échangeur d'anions consiste à séparer le galactanne par élution à l'eau. La fraction intéressante, constituée d'oses neutres, est repérée par détection spectrophotométrique à 200 nm et à 492 nm après coloration au test phénol-acide sulfurique, selon la méthode de DUBOIS (Anal. Chem. (1956) 28 ,

p. 350).

- Ladite fraction, constituée d'oses neutres, est soumise à une filtration sur gel qui permet de récupérer la fraction contenant du galactanne de poids moléculaire compris entre 4000 et 10000. La filtration sur gel est effectuée sur des supports commercialisés, par exemple sur Sephadex ou Biogel agarose ; on utilise de préférence Biogel A-1.5M.

La présente invention a également pour objet le galactanne obtenu par le procédé décrit ci-dessus.

Le galactanne extrait de Klebsiella, objet de la présente invention, possède de très intéressantes propriétés pharmacologiques ; il est doué notamment de remarquables propriétés immunostimulantes ainsi que d'une bonne tolérance. Il possède notamment la propriété de stimuler la production d'Il₁ et de TNF au niveau des macrophages et surtout de stimuler la génération de radicaux libres (polynucléaires). Il permet également de synergiser l'effet du GM-CSF (granulomonocyte-Colony Stimulating Factor).

Ces propriétés sont illustrées plus loin dans la partie expérimentale.

Ces propriétés justifient l'utilisation du galactanne extrait de Klebsiella tel que défini ci-dessus à titre de médicaments.

Ces médicaments trouvent, par exemple, leur emploi dans le traitement ou la prévention chez l'homme des immunodépressions, des maladies infectieuses causées par les bactéries ou les virus, dans le traitement des maladies à parasites, des toxi-infections, dans le traitement des infections posthospitalières et postchirurgicales et des allergies de toutes origines. Ces médicaments peuvent également être utilisés très avantageusement dans le traitement des greffes de moelle osseuse et des aplasies médullaires post-chimiothérapiques.

La dose usuelle, variable selon le produit utilisé, le sujet traité et l'affection en cause, peut être par exemple de 0,5 à 5 mg par jour, par voie orale.

L'invention a également pour objet les compositions pharmaceutiques qui renferment le galactanne extrait de Klebsiella tel que défini ci-dessus, à titre de principe actif.

A titre de médicaments, le galactanne extrait de Klebsiella tel que défini ci-dessus, peut être incorporé dans des compositions pharmaceutiques destinées à la voie digestive, parentérale ou locale.

Les compositions pharmaceutiques correspondantes peuvent être, par exemple, solides ou liquides et se présenter sous les formes pharmaceutiques couramment utilisées en médecine humaine, comme par exemple, les comprimés, simples ou dragéifiés, les gélules, les granulés, les solutions, les sirops, les suppositoires, les préparations injectables lyophilisées ou non, les ovules, les crèmes, les pommades, les lotions, les gouttes, les collyres, les aérosols ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Il va être donné maintenant à titre non limitatif, des exemples de mise en oeuvre de l'invention.

## Exemple 1 : préparation du galactanne .

On solubilise 334 mg d'acylglycannes de Klebsiella (obtenus comme indiqué à l'exemple 1 de la demande française n° 2 574 429 à partir de la souche déposée à l'Institut Pasteur sous le n° I-163) dans 33,4 cm³ de solution d'acide acétique à 1% et l'on chauffe à 100°C pendant 90 minutes. Après refroidissement, on sépare le précipité contenant la fraction lipidique par centrifugation à 2000 g pendant 30 minutes. On lyophilise le surnageant et isole 200 mg de résidu que l'on redissout dans 4 cm³ d'eau. On chromatographie, par fractions de 1 cm³, sur une colonne pour chromatographie à haute performance d'échangeur d'anions Whatman Magnum 9 SAX (9,4 mm *50 cm), en éluant par l'eau pendant 30 minutes, sous un débit de 2 cm³/minute en détectant à 200 nm. On isole la fraction renfermant les oses neutres repérées à 492 nm, après le test au phénol-sulfurique. On isole par lyophilisation 55 mg de fraction neutre.

## Exemple 2 : isolement du galactanne.

Le lyophilisat précédent est dissous dans du tampon eau-acide acétique-pyridine (973-7-20), puis on soumet à une filtration sur gel par chromatographie sur colonne de Biogel A.1.5M (2 cm *1.5m), équilibrée avec le même tampon et en éluant sous un débit de 12 cm³/heure avec le même tampon. On recueille la fraction détectée au test phénol-sulfurique. On stérilise par filtration sur membrane 0.22 $\mu$, puis on lyophilise et obtient 22 mg du galactanne attendu.

**Exemple 3 :**

On a préparé des comprimés répondant à la formule :

| | |
|---|---|
| - Produit de l'exemple 2 | 1 mg |
| - Excipient q.s. pour un comprimé terminé à | 100 mg. |

(Détail de l'excipient : lactose, amidon, talc, stéarate de magnésium).

**Exemple 4 :**

On a préparé des aérosols délivrant des doses contenant chacune :

| | |
|---|---|
| - Produit de l'exemple 2 | 0,5 mg |
| - Emulsifiant | 0,15 mg |
| - Propulseur | 50 mg. |

**Exemple 5 :**

On a préparé une crème répondant à la formule :

| | |
|---|---|
| - Produit de l'exemple 2 | 1 mg |
| - Excipient : alcool 2-octyl-dodécanol, alcool cétostéarylique, cétostéaryl sulfate de sodium, parahydroxybenzoate de méthyle et de propyle, eau purifiée | 10 mg. |

**ETUDE PHARMACOLOGIQUE**

**1.- Activation de polymorphonucléaires in vitro.**

Méthode :

On utilise la technique de chimiluminescence décrite par B. DESCAMPS-LATSCHA <ann. immunol Institut Pasteur (1982) 1330, 349-364> pour évaluer l'activation du métabolisme oxydatif phagocytaire des polynucléaires péritonéaux élicités chez la souris au thioglycolate, puis isolés par centrifugation à 500 G, par incubation in vitro avec le galactanne suivie d'une incubation avec le zymozan A.

Résultats :

Par incubation de $1.5 \times 10^6$ cellules par ml, pendant 20 minutes à 37°C avec le galactanne à tester à la concentration de 1 à 100 $\mu$g/ml, puis 10 minutes à 37°C avec le zymozan A à la concentration de 2 g/litre, on mesure le rapport de stimulation propre du galactanne, parallèlement à celui de lipopolysaccharide de Klebsiella.

TABLEAU I

|  | Concentration | RS |
|---|---|---|
| galactanne | 1 µg/ml | 0,96 |
|  | 10 | 0,94 |
|  | 100 | 2,30 |
| LPS | 0,4 ng/ml | 1,20 |
| de | 4 | 0,80 |
| Kb | 40 | 1,06 |

**2.- Etude sur les cellules médullaires totales.**

$10^5$ cellules de moelle osseuse sont mises en culture en agar en présence de $10^{-4}$ mg/ml de GM-CSF humain avec ou sans galactanne (produit obtenu à l'exemple 2).

Les colonies granulocytaires monocytaires sont comptées après 14 jours d'incubation à 37°C. Le nombre de colonies indiqué est la moyenne de 2 boîtes.

Les résultats obtenus figurent dans le tableau II ci-après.

TABLEAU II

|  | To | Galactanne | GM-CSF | GM-CSF + Galactanne | | |
|---|---|---|---|---|---|---|
|  |  |  |  | 0,1 µg | 1 µg | 10 µg |
| Exp.1 | 0 | 0 | 22 | 31 | 33,5 | 36 |
| Exp.2 | 0 | 0 | 33,5 | 42 | 55 | 32 |

Les résultats obtenus montrent que l'on obtient une augmentation très significative du nombre des colonies après addition de galactanne.

**3.- Etude sur des cellules mononuclées de moelle osseuse humaine.**

Les cellules mononuclées de moelle osseuse sont séparées par Ficoll puis ensemencées au nombre de $2.10^4$ cellules par millilitre de culture.

Les résultats obtenus figurent dans le tableau III ci-après.

TABLEAU III

|  | To | Galactanne | GM-CSF | GM-CSF + Galactanne | | |
|---|---|---|---|---|---|---|
|  |  |  |  | 0,1 $\mu$g | 1 $\mu$g | 10 $\mu$g |
| Exp.3 | 0 | 0 | 14,5 | 18,5 | 17,5 | 17 |

Les résultats obtenus montrent que l'on obtient une augmentation significative du nombre des colonies en présence de GM-CSF + 0,1 $\mu$g de galactanne par rapport au GM-CSF seul.

**Revendications**

1.- Le galactanne extrait de Klebsiella, caractérisé en ce qu'il est essentiellement constitué d'oses neutres.

2.- Le galactanne selon la revendication 1, caractérisé en ce qu'il est constitué d'un enchaînement linéaire de galactose en 1-3 et a un poids moléculaire compris entre 4000 et 10000.

3.- Le galactanne selon les revendications 1 ou 2, caractérisé en ce qu'il provient de Klebsiella pneumoniae.

4.- Le galactanne selon la revendication 3, caractérisé en ce qu'il provient de la souche déposée à l'Institut Pasteur à Paris ou Collection Nationale de Culture de Microorganismes sous les nupéros 52145 et I-163 et à la National Culture Type Collection sous le n° 5055.

5.- Procédé d'obtention du galactanne, tel que défini à l'une des revendications 1 à 4, caractérisé en ce que l'on traite un acylglycanne extrait de Klebsiella, essentiellement constitué d'environ 80% d'oses neutres, 20% de lipides, moins de 2% de protéines et ayant un poids moléculaire d'environ 12500, par hydrolyse acide douce, chromatographie à haute performance sur échangeur d'anions, récupère la fraction non retenue, soumet à une filtration sur gel, récupère la fraction contenant les galactannes de poids moléculaire compris entre 4000 et 10000.

6.- Procédé d'obtention du galactanne, tel que défini à l'une des revendications 1 à 4, dans lequel on prépare un acylglycanne à partir d'un extrait bactérien hydrosoluble de Klebsiella par chauffage puis fractionnement chromatographique, caractérisé en ce que l'on traite ledit acylglycanne par hydrolyse acide douce, chromatographie à haute performance sur échangeur d'anions, récupère la fraction non retenue, soumet à une filtration sur gel, récupère la fraction contenant les galactannes de poids moléculaire compris entre 4000 et 10000.

7.- Procédé selon la revendication 5 ou 6, caractérisé en ce que :
- l'hydrolyse acide douce est effectuée dans une solution à 1% d'acide acétique par chauffage à 100°C pendant 90 minutes,
- la chromatographie à haute performance sur échangeur d'anions consiste à séparer le galactanne par élution à l'eau.

8.- Le galactanne obtenu par le procédé selon l'une des revendications 5, 6 ou 7.

9.- Médicaments, caractérisés en ce qu'ils sont constitués par le galactanne tel que défini à l'une quelconque des revendications 1 à 4.

10.- Médicaments, caractérisés en ce qu'ils sont constitués par le galactanne tel que défini à la revendication 8.

11.- Compositions pharmaceutiques, caractérisées en ce qu'elles renferment, à titre de principe actif, l'un au moins des médicaments tels que définis à l'une quelconque des revendications 9 ou 10.

Revendications pour les Etats contractants suivants: ES, GR

1.- Procédé pour préparer le galactanne extrait de Klebsiella, essentiellement constitué d'oses neutres, caractérisé en ce que l'on traite un acylglycanne extrait de Klebsiella, essentiellement constitué d'environ 80% d'oses neutres, 20% de lipides, moins de 2% de protéines et ayant un poids moléculaire d'environ 12500, par hydrolyse acide douce, chromatographie à haute performance sur échangeur d'anions, récupère la fraction non retenue, soumet à une filtration sur gel, récupère la fraction contenant les galactannes de poids moléculaire compris entre 4000 et 10000.

2.- Procédé d'obtention du galactanne, tel que défini à la revendication 1, dans lequel on prépare un acylglycanne à partir d'un extrait bactérien hydrosoluble de Klebsiella par chauffage puis fractionnement chromatographique, caractérisé en ce que l'on traite ledit acylglycanne par hydrolyse acide douce, chromatographie à haute performance sur échangeur d'anions, récupère la fraction non retenue, soumet à une filtration sur gel, récupère la fraction contenant les galactannes de poids moléculaire compris entre 4000 et 10000.

3.- Procédé selon la revendication 1 ou 2, caractérisé en ce que :

- l'hydrolyse acide douce est effectuée dans une solution à 1% d'acide acétique par chauffage à 100°C pendant 90 minutes,

- la chromatographie à haute performance sur échangeur d'anions consiste à séparer le galactanne par élution à l'eau.

4.- Procédé selon l'une des revendications 1 à 3, caractérisé en ce que l'on utilise au départ un extrait bactérien hydrosoluble de Klebsiella Pneumoniae.

5.- Procédé selon la revendication 4, caractérisé en ce que l'on utilise au départ un extrait bactérien hydrosoluble provenant de la souche déposée à l'Institut Pasteur à Paris ou Collection Nationale de Culture de Microorganismes sous les nupéros 52145 et I-163 et à la National Culture Type Collection sous le n° 5055.

6.- Procédé de préparation de compositions pharmaceutiques, caractérisé en ce que l'on met à titre de principe actif l'un au moins des produits de formule (I) telle que définie à la revendication 1 sous une forme destinée à cet usage.

7.- Procédé de préparation de compositions pharmaceutiques, caractérisé en ce que l'on met à titre de principe actif l'un au moins des produits de formule (I) telle que définie à l'une quelconque des revendications 2 à 5, sous une forme destinée à cet usage.